Europäisches Patentamt

European Patent Office

Office européen des brevets

(11) Numéro de publication: **0 225 831 B1**

# FASCICULE DE BREVET EUROPEEN

(45) Date de publication de fascicule du brevet:
13.03.91

(51) Int. Cl.⁵: **A61K 35/78, A61K 37/22**

(21) Numéro de dépôt: **86402677.8**

(22) Date de dépôt: **02.12.86**

(54) Utilisation d'huiles hyperoxygénées pour la fabrication d'un médicament pour le traitement de l'herpès.

(30) Priorité: 06.12.85 FR 8518085

(43) Date de publication de la demande:
16.06.87 Bulletin 87/25

(45) Mention de la délivrance du brevet:
13.03.91 Bulletin 91/11

(84) Etats contractants désignés:
**AT BE CH DE ES GB GR IT LI LU NL SE**

(56) Documents cités:
EP-A- 0 117 962
FR-A- 2 461 744
FR-M- 2 330

(73) Titulaire: **Desjonqueres, Stéphane**
**7 rue du Chant des Oiseaux**
**F-78360 Montesson (Les Yvelines)(FR)**

(72) Inventeur: **Desjonqueres, Stéphane**
**7 rue du Chant des Oiseaux**
**F-78360 Montesson (Les Yvelines)(FR)**

(74) Mandataire: **Herrburger, Pierre**
**Cabinet Pierre Herrburger 115, boulevard**
**Haussmann**
**F-75008 Paris(FR)**

Rank Xerox (UK) Business Services

## Description

La présente invention se rapporte à l'utilisation de corps gras peroxydés pour la fabrication d'une composition médicamenteuse destinée au traitement de l'herpès.

L'herpès est une maladie d'origine virale sans thérapeutique étiologique connue aujourd'hui à part le ZOVIRAX à PARIS dont la composition est la suivante :

pour 100 g : Acyclovir D.C.I. ......... 3 g

Vaseline ................q.s.

dont l'efficacité n'est pas toujours évidente ; il s'attaque soit à la peau, soit aux muqueuses de la face (virus herpétique n° 1) soit à la peau et aux muqueuses génitales (herpes n° 2) et a un facteur de recurrence quasi systématique. Les poussées herpétiques reprennent à des intervalles souvent réguliers : tous les mois, de un à quelques jours, jusqu'à plus d'une année. Le facteur déclenchant ces crises est souvent d'origine émotive ; il peut également être physiologique comme la menstruation. L'herpès peut également avoir d'autres causes extérieures comme l'exposition au soleil (herpès solaire). L'herpès se caractérise par la présence de pustules douloureuses, ouvertes et suintantes, la plupart du temps accompagnées d'un caractère inflammatoire très marqué.

On n'a pas, jusqu'à ce jour, trouvé de traitement satisfaisant contre l'herpès et les seules thérapeutiques connues sont à visée symptomatique et ne permettent fréquemment pas de lutter efficacement contre la douleur herpétique.

Parmi les traitements de l'herpès actuellement proposés, on peut citer l'emploi d'anesthésiants de contact comme le Tronothane des Laboratoires Abbot qui a une action apaisante dont l'effet s'émousse avec la répétition des applications.

Conformément à l'invention, on s'est aperçu, de manière surprenante, que certains corps gras peroxydés appartenant à la famille des huiles naturelles hyperoxygénées possédaient des qualités propres leur permettant de se montrer nettement supérieurs aux compositions utilisées jusqu'à présent pour le traitement de l'herpès.

La possibilité d'utiliser des peroxydes de corps gras pour le traitement de certaines affections a été mentionnée pour la première fois dans le brevet français n° 2 330 M ; l'utilisation des compositions décrites dans ce document n'a, malheureusement, pas permis d'aboutir aux résultats espérés, notamment à cause de problèmes de tolérance, et pour cette raison, les recherches visant à améliorer ces compositions ont été interrompues pendant un certain nombre d'années.

Ces recherches ont été reprises récemment, en liaison avec l'intérêt accru du public pour les produits naturels, et en particulier, pour la médecine dite "douce" ; le brevet français n° 2 461 744 décrit, par exemple, un procédé de peroxydation de matières grasses pouvant aboutir à la préparation de composi tions utilisables dans le domaine médical.

On a ainsi, conformément à l'invention pu mettre en évidence des qualités pharmacologiques des huiles hyperoxygénées pouvant déboucher sur leur utilisation pour le traitement de l'herpès.

A cet effet, l'invention concerne l'utilisation, pour la fabrication d'une composition médicamenteuse destinée au traitement de l'herpès, d'au moins une huile naturelle d'origine végétale hyperoxygénée par saturation en oxygène et exposition intensive et contrôlée aux ultraviolets de façon à présenter un taux de peroxydation compris entre 30 et 300 exprimé en milli-équivalents et une teneur en glycérides oxydés comprise entre 5 et 40, cette huile étant essentiellement exempte de radicaux libres cytotoxiques et sa définition en spectrophotométrie UV étant de 1 à 6 pour le facteur E 270 et de 8 à 60 pour le facteur E 232. (NF T 60 223).

Le document EP-A 117 962 décrit également un procédé de peroxydation d'huiles végétales, mais les corps gras obtenus par la mise en oeuvre de ce procédé ne sont pas soumis à une exposition intensive et contrôlée aux ultraviolets et sont donc fondamentalement différents des corps gras utilisés conformément à l'invention.

Les huiles utilisées conformément à l'invention ont démontré une excellente action symptomatique sur l'herpès par une triple intervention sur les phénomènes douloureux inflammatoires et cicatrisants ; elles semblent avoir également une action sur la durée de la poussée herpétique.

L'activité anti-inflammatoire a été mise en évidence à partir d'un protocole expérimental traditionnel d'irritation locale de l'oreille du rat avec l'huile de croton ; au cours de ces expériences, l'activité de la composition objet de l'invention a été comparée avec celle de l'indométacine qui est l'anti-inflammatoire utilisé classiquement ; cette étude réalisée chez le rat a permis de conclure que l'administration topique de la composition, objet de l'invention, était capable de limiter de façon nette la phlogose induite localement par un agent irritant.

L'activité antalgique de cette composition a pu être démontrée par massage de témoins avec des huiles hyperoxygénées conformes à l'invention dans le cadre d'études cliniques en double aveugle, où le produit de comparaison était un anesthésiant de contact clairement défini par la pharmacopée française.

Des études cliniques réalisées en double aveugle contre placebo et d'autres travaux réalisés en ouvert, ont permis de mettre en lumière une activité cicatrisante indéniable, commune à toutes les huiles d'origine végétale hyperoxygénées répondant à la définition ci-dessus.

Selon une autre caractéristique de l'invention, on utilise une ou des huile(s) hyperoxygénée(s) choisie(s) dans le groupe formé par l'huile d'arachide, l'huile d'amande douce et l'huile de carthame.

Il a été trouvé que ces huiles présentent à un degré élevé, la triple activité anti-inflammatoire, antalgique et cicatrisante susmentionnée, et, en même temps se distinguent par une excellente tolérance et une absence totale de toxicité.

En effet, tous les travaux effectués dans ce sens (détermination de l'indice d'irritation primaire cutanée chez le lapin, détermination de l'indice d'irritation occulaire, étude de la toxicité transmuqueuse chez le lapin, recherche d'éventuels pouvoirs allergènes...) ont amené sans exception à conclure par un constat d'absence totale d'irritation cutanée ou de tout autre tolérance allergique ou non, qu'il s'agisse de la peau ou des muqueuses.

En pharmacologie humaine, tous les travaux cliniques entrepris ont confirmé sans exception les facteurs de tolérance mis en évidence par les travaux réalisés sur l'animal.

Parmi les huiles hyperoxygénées mentionnées ci-dessus, on a trouvé que l'huile de carthame hyperoxygénée possédait des propriétés antalgiques et anti-inflammatoires en faisant un produit particulièrement actif dans le traitement de l'herpès.

L'oxygène présent dans la composition et libéré dans le derme, qui est constitué uniquement d'oxygène non toxique (c'est-à-dire dépourvu de radicaux libres) pourrait agir au niveau des terminaisons nerveuses dermiques en empêchant le processus douloureux de se former, celui-ci semblant résulter de l'anoxie au niveau des récepteurs épidermiques de la douleur.

Bien entendu, conformément à l'invention, on peut n'utiliser qu'une ou plusieurs huiles naturelles d'origine végétale hyperoxygénées répondant à la définition mentionnée ci-dessus ; cependant, et selon une autre caractéristique de l'invention, on utilise avantageusement une composition se présentant sous la forme de gel.

A titre d'exemple, un tel gel peut contenir entre 88 et 93 % d'huile de carthame hyperoxygénée, entre 5 et 9 % d'un excipient à base d'huile d'amande douce et entre 1,5 et 3,5 % de parfum.

Le gel ayant la composition suivante :
- Huile de carthame hyperoxygénée ......... 90,7 %
- AEROSIL 300 (marque déposée) ............ 6,8 %
- Parfum ................................. 2,5 %

a permis d'obtenir des résultats particulièrement satisfaisants.

L'AEROSIL 300 est un excipient composé de silice commercialisé par la Société DEGUSSA FRANCE.

Il est remarquable de constater que les huiles hyperoxygénées, utilisées conformément à l'invention, sont dénuées de présence de radicaux libres cytotoxiques qui sont généralement associés à tout corps oxydé ; cette absence est de nature à justifier l'emploi de ces compositions en médecine humaine ; en effet, l'agressivité des radicaux libres sur la peau est bien connue et leur contribution à l'apparition de cancer a été fréquemment mentionnée.

Il est, en outre, essentiel de mentionner que l'invention n'est pas limitée à l'utilisation des huiles susmentionnées, et que toutes les huiles végétales peroxydées répondant à la définition ci-dessus peuvent parfaitement être utilisées dans le cadre de l'invention.

L'activité des huiles utilisées conformément à l'invention sera mise en lumière grâce à l'exemple ci-dessous qui est un compte-rendu d'expérience permettant d'étudier l'efficacité clinique et la tolérance cutanée d'une composition à base d'huile de carthame hyperoxygénée chez des malades présentant une poussée herpétique.

Au cours de cet essai, on a étudié l'efficacité clinique et la tolérance cutanée chez 32 sujets présentant une poussée d'herpès ; il s'agissait de 21 hommes et 11 femmes, dont l'âge variait de 18 à 82 ans. Tous les sujets présentaient un herpès récidivant de localisation cutanée dans 13 cas et muqueuse dans 19 cas. 25 sujets présentaient des signes fonctionnels à type de brûlure ou de prurit. La composition conforme à l'invention a été appliquée localement quatre à six fois par jour, pendant la durée d'évolution des lésions. Cette composition a été appliquée précocement dès l'apparition de l'éruption herpétique chez 20 malades, alors que les 12 autres ont été traités de façon plus tardive, au cours de l'évolution de la poussée d'herpès.

Sur l'ensemble des malades les résultats globaux ont fait apparaître 81 % de résultats satisfaisants. La composition s'est montrée active sur l'étendue de la lésion qui a été inférieure à celle notée au cours des poussées antérieures, dans la majorité des cas. De plus, la composition a nettement diminué la durée totale des poussées par rapport à leur durée habituelle ; la durée moyenne des poussées était de 13 jours lors des poussées précédentes ; elle est tombée à 9,17 jours lorsque le traitement a été entrepris au cours de la poussée d'herpès et à 4,11 jours lorsque la composition a été appliquée de manière plus précoce.

Enfin, la tolérance cutanée a été excellente, et on n'a noté qu'un seul cas d'eczématisation chez un malade de 71 ans présentant un herpès balanopréputial.

De façon plus précise, en étudiant les résultats en fonction des deux groupes précédemment détaillés soit :
- 12 sujets traités en cours d'évolution (groupe A)
- 20 sujets traités précocement (groupe B),

on a obtenu les résultats suivants :

| RESULTATS | GROUPE A | GROUPE B |
|-----------|----------|----------|
| Très bons | 7 cas | 13 cas |
| Bons | 2 cas | 3 cas |
| Notables | 1 cas | 1 cas |
| Nuls | 2 cas | 2 cas |

Intolérance : 1 cas.

Soit :
- dans le groupe A : Bons et très bons résultats: 75 %
- dans le groupe B : Bons et très bons résultats: 84 %

Les résultats en fonction de la localisation et des deux groupes susmentionnés sont les suivants :

Atteinte

| LOCALISATION | Très Biens | Biens | Notables | Nuls |
|---|---|---|---|---|
| Lèvres <br> 6 cas | Groupe A 1 cas <br> Groupe B 3 cas | | Groupe B 1 cas | Groupe B 1 cas |
| Gland <br> 2 cas | Groupe A 1 cas | Groupe B 1 cas | | |
| Région Balanopréputiale <br> 10 cas | Groupe A 3 cas <br> Groupe B 3 cas | Groupe A 1 cas <br> Groupe B 1 cas | Groupe A 1 cas | Groupe B 1 cas |
| Nez <br> 6 cas | Groupe A 3 cas <br> Groupe B 3 cas | | | |
| Pubis <br> 2 cas | Groupe B 1 cas | | | Groupe A 1 cas |
| Bourses <br> 1 cas | Groupe B 1 cas | | | |
| Fesses. Région interfessières. Région sacrée <br> 4 cas | Groupe B 2 cas | Groupe B 1 cas | | Groupe A 1 cas |

(Atteinte : MUQUEUSES / CUTANEES)

Pour mémoire : Intolérance : 1 cas (groupe B)

Le tableau précédent a permis de montrer que, dans les herpès muqueux, on a obtenu 74 % de résultats très satisfaisants, et, dans les herpès cutanés, 85% de résultats très satisfaisants.

Au total, les expériences ci-dessus ont permis de conclure que la composition utilisée conformément à l'invention a permis de soulager 3 malades sur 4 de leur prurit ou de leur brûlure en moins de 20 minutes, après son application, de limiter l'étendue et l'intensité des lésions d'en plus de 80 % des cas et de diminuer jusqu'à 70 % la durée d'évolution de la poussée.

L'invention propose donc l'utilisation d'une composition médicamenteuse parfaitement tolérée, exempte de toute toxicité, qui permet l'automédication.

Par comparaison, les produits d'allopathie par massage qui se prévalent d'un effet antiinflammatoire : cortisoniques et anti-inflammatoires non stéroïdiens (A.I.N.S.) sont tous soumis à la législation sur les produits toxiques dont la délivrance au malade exige une prescription médicale.


**Revendications**

1. Utilisation, pour la fabrication d'une composition médicamenteuse destinée au traitement de l'herpès, d'au moins une huile naturelle d'origine végétale, hyperoxygénée par saturation en oxygène et exposition intensive et contrôlée aux ultraviolets de façon à présenter un taux de peroxydation compris entre 30 et 300 exprimé en milli-équivalents et une teneur en glycérides oxydés comprise entre 5 et 40, cette huile étant essentiellement exempte de radicaux libres cytotoxiques et sa définition en spectrophotométrie UV étant de 1 à 6 pour le facteur E 270 et de 8 à 60 pour le facteur E 232.

2. Utilisation, selon la revendication 1, d'au moins une huile hyperoxygénée choisie dans le groupe formé par l'huile d'arachide, l'huile d'amande douce et l'huile de carthame pour la fabrication d'une composition médicamenteuse destinée au traitement de l'herpès.

3. Utilisation, selon l'une quelconque des revendications 1 et 2, d'huile de carthame pour la fabrication d'une composition médicamenteuse destinée au traitement de l'herpès.

4. Utilisation selon l'une quelconque des revendications 1 à 3, caractérisée en ce que la composition se présente sous la forme d'un gel.

5. Utilisation selon la revendication 4, caractérisée en ce que la composition contient entre 88 et 93 % d'huile de carthame hyperoxygénée, entre 5 et 9 % d'un excipient à base d'huile d'amande douce et entre 1,5 et 3,5 % de parfum.


## Claims

1. Use, for the manufacture of a medicinal composition intended for the treatment of herpes, of at least one natural oil of vegetable origin, hyperoxygenated by saturation with oxygen and intensive and controlled exposure to the ultraviolet so as to exhibit a degree of peroxidation of between 30 and 300 expressed in milli-equivalents and an oxidized glycerides content of between 5 and 40, this oil being essentially free from cytotoxic free radicals and its definition in UV spectrophotometry being from 1 to 6 in the case of the E 270 factor and from 8 to 60 in the case of the E 232 factor.

2. Use, according to Claim 1, of at least one hyperoxygenated oil chosen from the group consisting of groundnut oil, sweet almond oil and safflower oil for the manufacture of a medicinal composition intended for the treatment of herpes.

3. Use, according to either of Claims 1 and 2, of safflower oil for the manufacture of a medicinal composition intended for the treatment of herpes.

4. Use according to any one of Claims 1 to 3, characterized in that the composition is in the form of a gel.

5. Use according to Claim 4, characterized in that the composition contains between 88 and 93 % of hyperoxygenated safflower oil, between 5 and 9 % of an excipient based on sweet almond oil and between 1.5 and 3.5 % of perfume.


## Ansprüche

1. Verwendung von zumindest einem natürlichen Öl pflanzlicher Herkunft, zur Herstellung einer medikamentösen Zusammensetzung zur Behandlung von Herpes, welches hyperoxydiert ist durch Sättigung in Sauerstoff und intensiver und gesteuerter Aussetzung im Ultravioletten, um einen Peroxydationsgehalt zwischen 30 und 300, ausgedrückt in milli-Äquivalenten, und einen Gehalt an oxydierten Glyzeriden zwischen 5 und 40 aufzuweisen, wobei dieses Öl im wesentlichen frei von freien cytotoxischen Radikalen ist und seine spektrophotometrische Charakteristik von 1 bis 6 für den Faktor E270 und von 8 bis 60 für den Faktor E232 liegt.

2. Verwendung, gemäß Anspruch 1, von mindestens einem hyperoxydierten Öl, welches ausgewählt ist aus der Gruppe bestehend aus Erdnußöl, Mandelöl und Distelöl zur Herstellung einer medikamentösen Zusammensetzung zur Behandlung von Herpes.

3. Verwendung, gemäß einem der Ansprüche 1 und 2, von Distelöl zur Herstellung einer medikamentösen Zusammensetzung zur Behandlung von Herpes.

4. Verwendung gemäß einem der Ansprüche 1 - 3, dadurch gekennzeichnet, daß die Zusammensetzung gelförmig ist.

5. Verwendung gemäß Anspruch 4, dadurch gekennzeichnet, daß die Zusammensetzung zwischen 88 und

6

93 % hyperoxydiertes Distelöl, zwischen 5 und 9 % einer Grundmasse auf Mandelölbasis und zwischen 1,5 und 3,5 % Parfum beinhaltet.